# EUROPÄISCHE PATENTANMELDUNG

(11) **EP 4 529 872 A1**
(43) Veröffentlichungstag der Anmeldung: **02.04.2025**
(21) Anmeldenummer: 23200465.5
(22) Anmeldetag: 28.09.2023
(51) Int. Cl.: A61B 18/02, A61B 18/00, A61B 90/00

(54) **GASDOSIEREINHEIT ZUR GASVERSORGUNG EINES KRYOINSTRUMENTS**

(71) Anmelder: Erbe Elektromedizin GmbH, 72072 Tübingen (DE)
(72) Erfinder: Hiller, Jürgen, 72581 Dettingen (DE); Guendisch, Kai, 72762 Reutlingen (DE)
(74) Vertreter: Rüger Abel Patentanwälte PartGmbB

(57) **Zusammenfassung**

Die erfindungsgemäße Gasdosiereinheit weist ein Druckregelmodul (18) mit einer Druckminderungseinrichtung (21) auf, die dazu eingerichtet ist, einen gewünschten Gasfluss (m) einzustellen, (insbesondere CO₂-Strom). Bei normaler Raumtemperatur reguliert die Steuereinrichtung (31) die Druckminderungseinrichtung (21) zur Einstellung eines gewünschten Drucks oder eines gewünschten Gasstroms. Ist die Raumtemperatur und/oder die Temperatur des angeschlossenen Gasspeichers (12) niedriger und reicht demzufolge der an dem Eingangsanschluss (15) der Gasdosiereinheit anstehende Gasdruck für den korrekten Betrieb des Druckregelmoduls und des Instruments nicht aus, aktiviert die Steuereinrichtung (31) einen in dem Gaspfad liegenden Kompressor (20), der den Druck vor dem Druckregelmodul (18) auf einen zum korrekten Betrieb des Druckregelmoduls (21) geeigneten Wert erhöht. Damit lässt sich das Kryoinstrument (11) unabhängig von der Raumtemperatur und weitgehend unabhängig von dem Druck in dem Druckspeicher (12) korrekt aktivieren und benutzen. Sonst bestehende Betriebseinschränkungen werden vermieden.

## Beschreibung

Die Erfindung betrifft eine Gasdosiereinheit, die zur Gasversorgung eines Kryoinstruments eingerichtet ist, sowie auch die Kombination aus Gasdosiereinheit, Gasspeicher und Kryoinstrument. Solche Kryoinstrumente können z.B. zur Biopsieentnahme oder auch für andere medizinische Zwecke, z.B. Kryoablation usw., verwendet werden.

Die erfindungsgemäße Gasdosiereinheit dient der Versorgung des Kryoinstruments mit einem gasförmigen Kältemittel, wie insbesondere CO₂, N₂O oder einem anderen zur Kälteerzeugung geeigneten insbesondere wenigstens 3-atomigen Gas. Das Kryoinstrumemnt arbeitet nach dem Kryoprinzip, wonach eine in Kontakt mit biologischem Gewebe befindliche Instrumentenspitze soweit abgekühlt wird, dass Gewebe an der Instrumentenspitze anfriert und haftet. So kann mit dem Instrument das an der Instrumentenspitze haftende Gewebe einem Patienten beispielsweise zur weiteren Untersuchung oder auch zu anderen Zwecken entnommen werden.

Zur Speisung einer Kryoablationseinrichtung offenbart die US 2022/0068929 A1 eine Gasspeiseeinrichtung mit einem Kompressionsgefäß, das eine flexible Wand aufweist, die den Innenraum des Kompressionsgefäßes in zwei Teilräume unterteilt. Durch Druckbeaufschlagung des einen Teilraums kann das in dem anderen Teilraum befindliche Medium unter Druck gesetzt und einem Gasverbraucher zugeführt werden.

Zur Versorgung von Kryoinstrumenten mit komprimiertem Gas und zur Kühlung von deren Spitze mittels des Joule Thomson Effekts offenbart die WO 2004/064914 A2 eine Gasdosiereinheit, bei der von Instrumenten mit niedrigem Druck zurückkehrendes Gas mittels eines Kompressors komprimiert und dann mit hohem Druck über sogenannte Strömungsregulatoren den Instrumenten wieder zugeführt wird.

Weiter offenbart die EP 3 854 334 A1 eine Gasdosiereinheit zur Gasversorgung eines Kryoinstruments. Die Dosiereinheit weist einen Eingangsanschluss für einen Gasspeicher und einen Ausgangsanschluss für das Instrument auf. Zwischen dem Eingangsanschluss und dem Ausgangsanschluss ist ein Druckregelmodul angeordnet. Dieses umfasst eine Pumpe, die zum Beispiel als doppelt wirkende Kolbenpumpe ausgebildet sein kann und dazu eingerichtet ist, Gas aus dem Druckspeicher in ein Puffergefäß zu fördern. Dem Puffergefäß ist eine Temperiereinrichtung zugeordnet, die zur Beheizung oder auch zur Kühlung des Puffergefäßes dienen kann. Außerdem weist das Druckregelmodul ein Druckregelventil auf, um gezielt und kontrolliert gasförmiges Kältemittel aus dem Puffergefäß an den Ausgangsanschluss und somit zu dem Instrument zu liefern. Eine Steuerungseinrichtung ist dabei dazu eingerichtet, den Druck und/oder die Temperatur in dem Puffergefäß zu erfassen und dessen Temperierung zu steuern.

Beim Einsatz von Kryoinstrumenten wird insbesondere bei der Biopsieentnahme häufig eine sehr schnelle Aktivierung der Sonde gewünscht, um die für die Biopsie erforderliche Zeit gering zu halten. Beispielsweise dauert eine übliche Anwendung einer Biopsiesonde nur 3 bis 5 s, sodass die Gefrierleistung an der Instrumentenspitze nach Aktivierung der Gasdosiereinheit möglichst sofort zur Verfügung stehen soll.

Als Kältemittel zum Betrieb des Kryoinstrumemnts soll in erster Linie ein Gas dienen, das bei Expansion einen starken Joule Thomson Effekt und somit eine ausgeprägte Kälteentwicklung zeigt. Ein solches Gas ist CO₂ oder auch N₂O. Auch andere drei- oder mehratomige Gase insbesondere mit polaren Molekülen sind gut anwendbar.

Typischerweise werden solche Gase Druckspeichern beispielsweise Gasflaschen oder dergleichen entnommen, in denen das Gas in durch Druck verflüssigter Form vorliegt. Dieses Gas wird dem Kryoinstrument typischerweise über ein Druckregelmodul zugeführt, wobei sich aber gezeigt hat, dass die Gefrierleistung des Kryoinstruments nach Aktivierung verzögert einsetzen kann, wenn die Temperatur des Gasspeichers, beispielsweise aufgrund niedriger Raumtemperatur oder aus anderen Gründen, eine Temperaturgrenze unterschreitet. Z.B. kann bei Raumtemperaturen unter etwa 18°C die Gefrierleistung an dem Kryoinstrument verzögert einsetzen oder unter der erforderlichen Leistung bleiben. Dies kann letztendlich die nötige Applikationsdauer verlängern oder die Anwendung ganz unmöglich machen, was von Behandlern abgelehnt wird. Dies betrifft insbesondere besonders lange und dünne Instrumente.

Daraus leitet sich die der Erfindung zugrundeliegende Aufgabe ab, eine Gasdosiereinheit zu schaffen, mit der das Kryoinstrumment auch bei niedrigen Temperaturen des Gasspeichers nach Aktivierung unverzögert mit der gewünschten Kälteleistung arbeitet.

Diese Aufgabe wird mit der Gasdosiereinheit nach Anspruch 1 gelöst:

Die erfindungsgemäße Gasdosiereinheit enthält ein Druckregelmodul, das zwischen einem Eingangsanschluss und einem Ausgangsanschluss der Gasdosiereinheit angeordnet ist. An den Eingangsanschluss ist ein Gasspeicher anschließbar, während an den Ausgangsanschluss ein Kryoinstrument anschließbar ist. Das Druckregelmodul ist dazu eingerichtet, Gas von dem Eingangsanschluss zu dem Ausgangsanschluss zu leiten, wobei das Gas dabei die Druckminderungseinrichtung durchfließt. Vor der Druckminderungseinrichtung ist mindestens ein Drucksensor angeordnet, um den Gasdruck des der Druckminderungseinrichtung zugeführten Gases zu erfassen und mit einem Grenzwert Pmin zu vergleichen. Die Steuereinrichtung ist dazu eingerichtet, den Kompressor zu aktivieren, um den Druck des der Druckminderungseinrichtung zugeführten Gases auf oder über den Grenzwert Pmin anzuheben, wenn der Druck des von dem Gasspeicher herkommenden Gases unterhalb des Grenzwerts Pmin liegt.

Der Kompressor ist mit einem Motor verbunden und von diesem angetrieben. Der Motor ist mit der Steuereinrichtung verbunden, die den Motor mit einer variablen von ihr vorgegebenen Drehzahl betreibt. Damit ist die zugleich mit mindestens einem Drucksensor verbundene Steuereinrichtung dazu eingerichtet, mittels des Kompressors einen ausreichend hohen Druck aufzubauen, damit die Druckminderungseinrichtung einen kontrollierten Gasfluss gewünschter Höhe zu dem Kryoinstrument liefert.

Weiter kann die Steuereinrichtung dazu eingerichtet sein, den Motor und mit ihm den Kompressor nur dann zu betreiben, d.h. zu aktivieren, wenn ein an dem Instrument oder separat angeordneter, mit der Steuereinrichtung verbundener Aktivierungsschalter betätigt wird. Damit kann sichergestellt werden, dass der Kompressor nur zur Aktivierung des Instruments, nicht aber in Pausenzeiten läuft. Dadurch ist es möglich, auf Puffervolumina zwischen dem Kopressor und dem nachfolgenden Ventil (Aktivierungsventil) zu verzichten und auch das dort vorhandene Leitungsvolumen zu minimieren. Dadurch wird andererseits ein schneller Druckaufbau bei Aktivierung des Kompressors erreicht. Auch trägt dies zur Geräuschminderung und dadurch zur Ruhe am Patienten bei. Außerdem werden für den Kompressor besonders ruhig laufende Exemplare bevorzugt, die dann mindestens zwei oder auch mehrere Kolben aufweisen. Es haben sich dabei langsamlaufende Kompressoren mit Maximaldrehzahlen unter 300 U/min als besonders laufruhig herausgestellt, die über ein etwa quadratisches Bohrungshubverhältnis verfügen und deren Pleuelstange wenigstens 5 mal, besser 7 bis 8 mal so lang ist wie der Kurbelradius ihrer Kurbelwelle. Der Kurbelradius ist mit der Exzentrizität des Hubzapfens seiner Kurbelwelle identisch.

Bei einer bevorzugten Ausführungsform weist das Kryoinstrument eine Gasrückführungsleitung auf, die an die Gasdosiereinheit angeschlossen ist. Die Gasdosiereinheit weist einen Gaspfad für das rückgeführte Gas auf, in welchem ein Massenflusssensor angeordnet ist. Dieser ist vorzugsweise mit dem Druckregelmodul verbunden, um den Druck des zu dem Kryoinstrument rückgeführten Gases auf einen Wert einzustellen, der zu einem gewünschten Massenfluss passt. Die Steuereinrichtung des Druckregelmoduls ist dazu eingerichtet, den gewünschten Massenfluss mit dem realen gemessenen Massenfluss zu vergleichen und den Druck des zu dem Instrument geführten Gases entsprechend zu erhöhen oder zu vermindern, um die gewünschte Gleichheit von gewünschtem Massenfluss und gemessenem Massenfluss herzustellen. Insbesondere bei sehr dünnen und/oder sehr langen Instrumenten kann es sein, dass der dazu erforderliche Druck deutlich höher ist als der von dem Gasspeicher bereitgestellte Druck. In diesem Fall ist die Steuereinrichtung 31 dazu eingerichtet den Kompressor zu aktivieren.

Bei einer möglichen anderen Ausführungsform weist das Druckregelmodul einen ersten Drucksensor zwischen dem Eingangsanschluss und dem Kompressor und einen zweiten Drucksensor zwischen dem Kompressor und der Druckminderungseinrichtung auf. Beide Drucksensoren sind vorzugsweise mit der Steuereinrichtung verbunden. Weiter kann die Druckminderungseinrichtung ein steuerbares Ventil aufweisen, dessen Antriebseinrichtung an die Steuereinrichtung angeschlossen ist.

Zwischen dem Ventil und dem Ausgangsanschluss oder, wie oben erwähnt in einer Gasrückführungsleitung, kann die Strömungsmesseinrichtung angeordnet sein. Die Strömungsmesseinrichtung kann zum Beispiel eine Drossel sowie vor und nach der Drossel angeordnete Drucksensoren umfassen. Andere Bauformen mit Flügelrad, Ultraschall- oder Wärmemessung sind möglich. Z.B. kann einer der Drucksensoren zwischen dem Ventil und der Drossel angeordnet sein während der andere Drucksensor zwischen der Drossel und dem Ausgangsanschluss angeordnet sein kann. Beide Drucksensoren können mit der Steuereinrichtung verbunden sein. Diese kann dazu eingerichtet sein, das Ventil so einzustellen, dass an der Drossel ein gewünschter Druckabfall und somit ein gewünschter Massenstrom vorhanden ist um das Kryoinstrument zu speisen.

Das von der Steuereinrichtung gesteuerte Ventil kann eine Schließstellung und eine Offenstellung sowie vorzugsweise dazwischenliegende Regelstellungen aufweisen, in denen es den durchfließenden Gasstrom mehr oder weniger drosselt. Damit ist das Ventil zum einen dazu eingerichtet, den Massenstrom des Gases zu dem Instrument konstant zu halten und dabei zum anderen auch Druckpulsationen zu beseitigen, die von dem Betrieb des Kompressors herrühren oder herrühren können.

Die Steuereinrichtung kann verschiedene Betriebsarten einnehmen. Nach Aktivierung durch die Betätigung eines Aktivierungsschalters kann die Steuereinrichtung zunächst überprüfen, ob der vor dem Ventil vorhandene Druck größer als der Mindestdruck ist. Dieser Druck kann mit dem ersten Drucksensor oder gegebenenfalls auch mit dem zweiten Drucksensor erfasst werden. Die Steuereinrichtung kann dazu eingerichtet sein, bei Unterschreitung des Mindestdrucks Pmin den Motor des Kompressors zunächst mit einer vorgegebenen, vorzugsweise der höchstmöglichen, Drehzahl zu betreiben. Die Steuereinrichtung kann dazu eingerichtet sein, diese Betriebsart für eine festgelegte Zeitspanne zum Beispiel 1 oder 2 s einzunehmen. Es ist jedoch auch möglich, diese Betriebsart abzubrechen sobald der mittels des zweiten Drucksensors überwachte Druck in der Leitung zwischen dem Kompressor und der Druckminderungseinrichtung einen Maximaldruck Pmax erreicht hat, um dann in die zweite Betriebsart zu wechseln. Während in der ersten Betriebsart die Drehzahl des Motors auf einem bestimmten Wert gehalten wird, kann die Steuereinrichtung dazu eingerichtet sein, die Drehzahl des Motors in der zweiten Betriebsart entsprechend dem gewünschten Druck in der Leitung zwischen dem Kompressor und der Druckminderungseinrichtung einzuregulieren. Damit steht der Druckminderungseinrichtung immer ein ausreichender Druck zur Verfügung, um das Kryoinstrument mit dem nötigen Massenstrom gasförmigen Kryofluids zu versorgen.

Die Steuereinrichtung kann dazu eingerichtet sein, eine dritte Betriebsart einzunehmen, in welcher der Motor nicht arbeitet und der Kompressor stillsteht. Die Steuereinrichtung kann dazu den von dem ersten und/oder dem zweiten Drucksensor erfassten Druck nutzen. Liegt der Druck des von dem Gasspeicher gelieferten Gases zwischen dem Mindestdruck Pmin und dem Maximaldruck Pmax, steht ein ausreichender Ausgangsdruck zum korrekten Betrieb der Druckminderungseinrichtung zur Verfügung. Der Gasstrom kann dann durch die Arbeitsräume des nicht betriebenen Kompressors (dessen Kolben dann unbeweglich stehen) oder durch einen dazu freigegebenen Bypass zu dem Ventil fließen, mittels dessen die Steuereinrichtung dann den gewünschten Gasstrom einreguliert.

Weitere Einzelheiten von Ausführungsformen der Erfindung sind Gegenstand der Zeichnung, sowie der zugehörigen Beschreibung oder von Unteransprüchen.

In der Zeichnung sind Ausführungsformen der Erfindung veranschaulicht. Es zeigen:
Figur 1 Eine Gasdosiereinheit mit einem angeschlossenen Kryoinstrument, in schematisierter Gesamtdarstellung,
Figur 2 die Gasdosiereinheit, in schematischer Darstellung,
Figur 3 ein Diagramm zur Veranschaulichung des Betriebs der Komponenten der Gasdosiereinheit,
Figur 4 den Kompressor und Teile der Druckminderungseinrichtung, in schematisierter Darstellung,
Figur 5 bis 8 alternative Bauformen der erfindungsgemäßen Gasdosiereinheit.

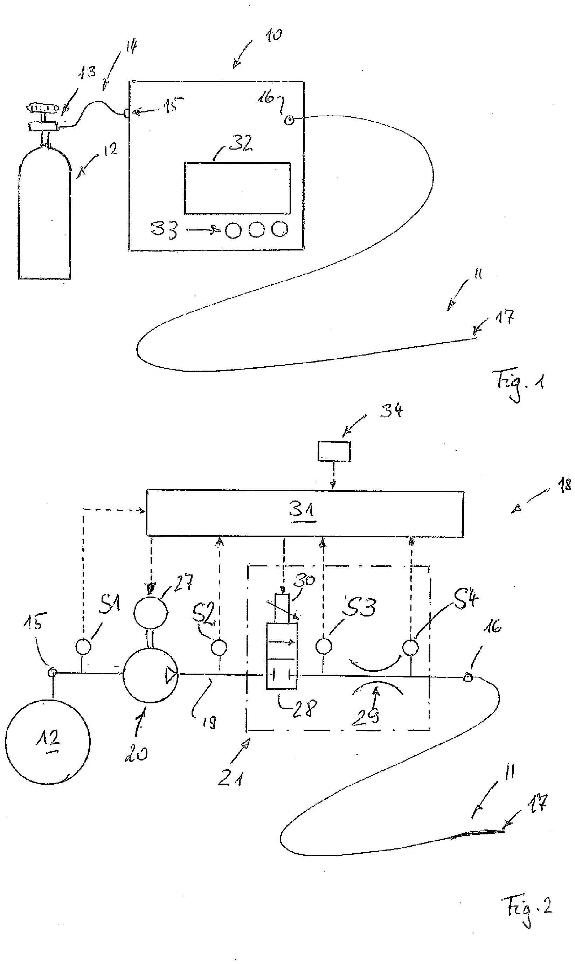

In Figur 1 ist eine Gasdosiereinheit 10 veranschaulicht, die dazu dient, ein Kryoinstrument 11 mit Gas aus einem Gasspeicher 12 zu versorgen. Der Gasspeicher 12 kann zum Beispiel eine Gasflasche sein, in der das zur Versorgung des Instruments 11 dienende Gas unter Druck verflüssigt bereitgehalten wird. Typische Gase sind dafür CO₂, N₂O oder andere mehratomige Gase mit einer Siedetemperatur deutlich unter 0° C. Der in dem Gasspeicher 12 vorhandene Druck liegt typischerweise zwischen 40 und 60 bar.

Der Gasspeicher 12 weist einen Anschluss 13 auf, von dem eine Leitung 14, vorzugsweise ohne Zwischenschaltung eines Druckminderungsventils, zu einem Eingangsanschluss 15 der Gasdosiereinheit 10 führt. Die Gasdosiereinheit 10 weist außerdem einen Ausgangsanschluss 16 auf, an dem das Kryoinstrument 11 angeschlossen ist und über den es mit Gas zur Kryokühlung seiner Spitze 17 versorgt wird.

Das Kryoinstrument 11 ist insbesondere ein langes, schlankes, flexibles Instrument mit einem Sondenschlauch, der an der Spitze 17 mit einem typischerweise aus Metall bestehenden Endstück geschlossen ist. In diesem Endstück mündet eine Kapillare, aus der das von der Gasdosiereinheit 10 gelieferte Gas expandierend ausströmt und somit die Spitze 17 des Kryoinstruments 11 von innen her mittels des Joule-Thomson-Effekts kühlt. Dabei werden Temperaturen deutlich unter 0° C erreicht, sodass Gewebe an der Spitze 17 anfrieren und fest anhaften kann.

Der Außendurchmesser des Kryoinstruments 11 kann weniger als 2 mm betragen. Entsprechend eng sind das darin vorhandene Lumen und die in dem Lumen angeordnete Kapillare. Die Länge des Kryoinstruments 11 kann deutlich über 1m liegen. Auch Außendurchmesser unter 1 mm und/oder Längen über 2 m sind möglich, wobei der Gasdruck des speisenden Gases ausreichend hoch sein muss, um die sich ergebenden Strömungswiderstände zu überwinden. Das Kryoinstrument mit einer Länge von wenigstens 1,5 m, 2 m, oder bis zu oder über 2,50 m eignet sich, insbesondere wenn es einen Außendurchmesser von weniger als 2 mm, oder weniger als 1 mm aufweist, zur Entnahme einer Gewebeprobe (Biopsie) im Gallengang eines Patienten. Die Erfindung erstreckt sich auch auf die Durchführung einer solchen Biopsie mit einem Kryoinstrument 11 und einer Gasdosiereinheit 10 der beschriebenen Bauart. Ein solches Kryoinstrument 11 kann besonders vorteilhaft an einer Gasdosiereinheit 10 der beschriebenen Bauart betrieben werden, die CO₂ (oder ein anderes geeignetes Gas) mit einem ausreichenden Druck liefert, der den Druck des Gasspeichers 12 übersteigen kann. Die Gasdosiereinheit 10 eignet sich insbesondere, die infolge der Länge und des geringen Durchmessers des Kryoinastruments 11 erhöhten inneren Strömungswiderstände zu überwinden und eine gute Kühlwirkung an seiner Spitze 17 zu erzielen. Solche schlanken Kryoinstrumente 11 können auch zur Biopsie an anderen Stellen des Körpers des Patienten in anderen engen Körperlumina eingesetzt werden.

Zur zuverlässigen Gasversorgung des Kryoinstruments 11 ist die Gasdosiereinheit 10 vorgehsehen, deren prinzipieller Aufbau aus Figur 2 hervorgeht. Sie enthält ein in Figur 2 veranschaulichtes Druckregelmodul 18, in dem ein sich von dem Eingangsanschluss 15 zu dem Ausgangsanschluss 16 erstreckender Gaspfad 19 ausgebildet ist, den das Gas von dem Eingangsanschluss 15 zu dem Ausgangsanschluss 16 durchströmt. Der Gaspfad 19 enthält einen Kompressor 20 und, auf diesen in Durchströmungsrichtung folgend, eine Druckminderungseinrichtung 21.

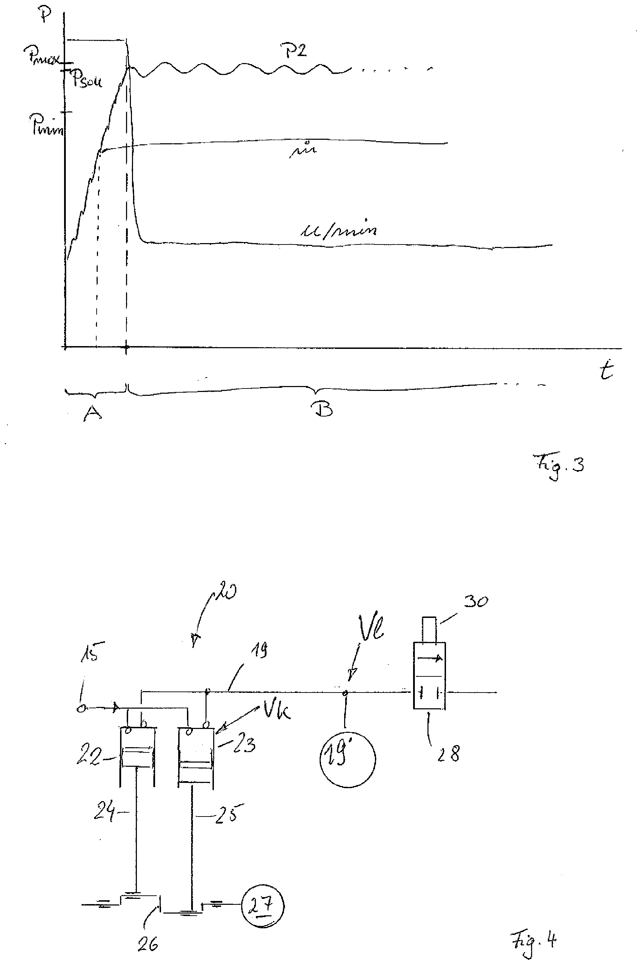

Der Kompressor 20 ist vorzugsweise ein Kolbenkompressor, weiter vorzugsweise ein Mehrkolbenkompressor, wie er schematisch in Figur 4 veranschaulicht ist. Seine beiden Zylinder 22, 23 enthalten Kolben, die über Pleuelstangen 24, 25 mit Hubzapfen einer Kurbelwelle 26 verbunden sind, die von einem Motor 27 mit variabler Drehzahl drehend antreibbar ist. Die Exzentrizität der Hubzapfen der Kurbelwelle 26 ist dabei vorzugsweise klein gegen die Länge der Pleuelstangen 24, 25, d.h. vorzugsweise kleiner als ein 5-tel oder 8-tel von deren Länge. Allerdings wird darauf hingewiesen, dass anstelle von Kolbenkompressoren auch andere Kompressoren, z.B. rotierende Verdrängerpumpen, Zahnradpumpen, Drehkolbenpumpen oder dergleichen Anwendung finden können.

Die beiden Kolben grenzen in den Zylindern 22, 23 ein Hubvolumen ab, das insgesamt zwischen ein und drei Kubikzentimeter liegen kann. Das sich anschließende Volumen Vl des Gaspfads 19 ist in Figur 4 gesondert durch das Druckspeichersymbol 19' veranschaulicht. Das Volumen Vl ist höchstens 5 vorzugsweise höchstens 3 bis 4 mal so groß wie dieses Hubvolumen des Kompressors 20. Damit wird sichergestellt, dass das Volumen Vl mit wenigen Umdrehungen der Kurbelwelle 16 gefüllt ist und den nötigen Druck aufweist.

Wie aus Figur 2 ersichtlich, sind in dem Gaspfad 19 mehrere Drucksensoren S1 bis S4 angeordnet. Ein erster Drucksensor S1 ist zwischen dem Eingangsanschluss 15 und dem Kompressor 20 angeordnet und liefert ein Signal, das einen ersten Druck P1 kennzeichnet. Ein zweiter Drucksensor S2 ist zwischen dem Kompressor 20 und der Druckminderungseinrichtung 21 angeordnet und liefert ein Signal, das einen zweiten Druck P2 kennzeichnet. Ein dritter Drucksensor S3 kann in der Druckminderungseinrichtung zwischen einem darin angeordneten Ventil 28 und einer darauffolgenden Drossel 29 angeordnet sein und ein Signal liefern, das einen dritten Druck P3 kennzeichnet. Zur Bestimmung des Massenflusses kann danach eine Drossel 29 und ein vierter Drucksensor S4 zwischen der Drossel 29 und dem Ausgangsanschluss 16 angeordnet sein. Der vierte Drucksensor S4 liefert ein Signal, das einen vierten Druck P4 kennzeichnet. Die Druckdifferenz P3 - P4 kennzeichnet den Massenstrom m. Zu der Druckminderungseinrichtung gehören bei dieser Ausführungsform das Ventil 28, die Drossel 29 und die beiden Drucksensoren S3, S4.

Das Ventil 28 kann als 2/2-Wege-Ventil ausgebildet und mit einem Stellantrieb 30 versehen sein, um zwischen der Schließstellung und der Offenstellung verschiedene Drosselstellungen einnehmen zu können. Es dient als Aktivierungsventil, um den Gasfluss freizugeben oder zu stoppen.

Die Drucksensoren S1 bis S4 sind mit einer Steuereinrichtung 31 verbunden, die außerdem dazu eingerichtet ist, den Motor 27 und den Stellantrieb 30 zu steuern. Die Steuereinrichtung 31 kann ein Rechner, ein Mikrocontroller oder eine andere digitale oder analoge Steuerschaltung sein, die aus einer oder mehreren Baugruppen besteht. Sie kann außerdem mit einer Anzeigeeinrichtung 32 (Figur 1) und einem oder mehreren Eingabemitteln 33 verbunden sein, die an einem Gehäuse der Gasdosiereinheit angeordnet sein können. Über die Anzeigeeinrichtung 32 und die Eingabemittel 33 können Betriebszustände angezeigt und vorgegeben werden.

Weiter ist die Steuereinrichtung 31 mit einem Aktivierungsschalter 34 verbunden (Figur 2), der ebenfalls an dem Gehäuse der Dosiereinheit 10, als gesonderter Schalter, beispielsweise als Fußschalter oder auch an dem Instrument angebracht sein kann. Letzteres insbesondere, wenn das Instrument als Handinstrument für den offenchirurgische Einsatz vorgesehen ist. Bei einem typischen Anwendungsfall ist das Kryoinstrument 11 jedoch als schlanke, flexible Sonde für den endoskopischen Einsatz vorgesehen und ausgebildet.

Die insoweit beschriebene Gasdosiereinheit 10 arbeitet im Zusammenspiel mit dem Kryoinstrument 11 und dem Gasspeicher 12 wie folgt:
Zur Inbetriebnahme der Gasdosiereinheit 10 wird zunächst der Gasspeicher 12 zum Beispiel mittels der Leitung 14 an den Eingangsanschluss 15 angeschlossen und die Gasdosiereinheit 10 in Betriebsbereitschaft versetzt. Die Gasdosiereinheit kann dabei als eigenständiges Gerät oder als Teil eines umfassenden größeren Geräts ausgebildet sein.

Nach dem Anschluss des Gasspeichers 12 an die Gasdosiereinheit 10 strömt aus dem Gasspeicher 12 Gas in den Gaspfad 19 bis zu dem geschlossenen Ventil 28, bis der von dem Drucksensor S1 aufgenommene Druck P1 im Wesentlichen dem Innendruck des Gasspeichers 12 entspricht. Das Gas kann außerdem durch die in Figur 4 schematisch durch kleine Kreise angedeuteten Rückschlagventile des Kompressors 20 bis zu dem Ventil 28 vordringen, sodass auch der Drucksensor S2 den entsprechenden Gasdruck P2 erfasst und ein entsprechendes Signal an die Steuereinrichtung 31 abgibt. Die Gasdosiereinheit 10 ist nun einsatzbereit.

Zur Biopsieentnahme oder sonstigen Behandlung wird das Kryoinstrument 11 an den Ausgangsanschluss 16 angeschlossen und mit seiner Spitze 17 an den Einsatzort geführt. Durch Betätigung des Aktivierungsschalters 34 wird das Kryoinstrument 11 aktiviert, d.h. es beginnt zu kühlen. Im Einzelnen überprüft die Steuereinrichtung 31 nach Empfang eines Aktivierungssignals von dem Aktivierungsschalter 34 zunächst den Druck P2 an dem Drucksensor S2. Ist dieser größer als ein zum korrekten Betrieb der Druckminderungseinrichtung 21 und des Kryoinstruments 11 mindestens erforderlichen Druck Pmin, bleibt der Kompressor 20 deaktiviert, d.h. die Steuereinrichtung 31 veranlasst den Motor 27, nicht zu drehen. Sie gibt außerdem ein Signal an den Stellantrieb 30, um das Ventil 28 zu öffnen. Es strömt nun Gas durch die Drossel 29 zu dem Kryoinstrument 11 (und von diesem über einen nicht weiter veranschaulichten Auslass zurück oder ins Freie). Dabei liefern die Drucksensoren S3 und S4 die den jeweiligen Druck P3, P4 kennzeichnenden Signale, aus denen die Steuereinrichtung 31 den über der Drossel 29 abfallenden Differenzdruck P3-P4 bestimmt. Um einen bestimmten Gasstrom (Liter pro Minute) zu dem Kryoinstrument sicherzustellen, beeinflusst die Steuereinrichtung 31 den Stellantrieb 30 so, dass dieser das Ventil 28 graduell weiter öffnet oder schließt. Die Steuereinrichtung 31 arbeitet in Verbindung mit den Drucksensoren S3 und S4 sowie dem Stellantrieb 30 als PID-Regler, bei beispielweise stehendem Kompressor 20.

Ein anderer Betriebsfall liegt vor, wenn die Temperatur im Operationssaal oder insbesondere die Temperatur des Gasspeichers 12 so niedrig liegt, dass der Gasdruck P1 und/oder P2 an dem Sensor S1 und/oder S2 den Mindestdruck Pmin unterschreitet. Dieser Betriebsfall ist in Figur 3 veranschaulicht. Unmittelbar nach Einleitung des Betriebsbeginns durch Betätigung des Aktivierungsschalters 34 stellt die Steuereinrichtung 31 durch Überprüfung des Drucks P1 und/oder S2 an dem Sensor S1 und/oder S2 fest, dass der vorhandene Gasdruck unter dem Mindestdruck Pmin liegt. Sie ist darauf eingerichtet, daraufhin den Motor 27 mit Maximaldrehzahl zu betreiben. In dieser Betriebsphase arbeitet die Steuereinrichtung 31 nicht wie sonst als PID-Regler, sondern sie überwacht lediglich den Druck P2 an dem Sensor S2, ohne dabei die Motordrehzahl zu reduzieren. Sobald der Druck an dem Sensor S2 jedoch den Maximaldruck Pmax oder auch einen etwas unter diesem liegenden, zum Betrieb des Druckreglers ausreichenden Druck Psoll erreicht, beendet die Steuereinrichtung 31 die Betriebsphase A und geht dazu über, den Druck P2 gemäß dem Solldruck Psoll zu regeln. Dabei reduziert die Steuereinrichtung 31 die Motordrehzahl und regelt diese als PID-Regler.

Aufgrund des relativ kleinen zwischen dem Kompressor 20 und dem Ventil 28 eingeschlossenen Volumens Vl im Verhältnis zum Hubvolumen des Kompressors sind, wie Figur 3 auch zeigt, die Druckschwankungen um den Sollwert Psoll herum noch relativ groß. Die Steuereinrichtung 31 ist aber dazu eingerichtet, diese Druckschwankungen mittels des Stellantriebs 30 in einer zweiten Regelschleife auszuregeln. Die Steuereinrichtung 31 bildet somit eine erste Regelschleife für den Druck, mit der die Kompressordrehzahl geregelt wird. Sie bildet außerdem eine zweite Regelschleife für das Ventil 28 zum Ausgleichen der auftretenden Druckschwankungen. Die Zeitkonstante der zweiten Regelschleife ist vorzugsweise wesentlich geringer als die Zeitkonstante der ersten Regelschleife. Damit ergibt sich hinter dem Ventil 28 an dem Drucksensor S3 ein glatter Druckverlauf ohne die Schwankungen, die der Kompressor 20 hervorruft. Entsprechend stellt sich ein konstanter Gasmassenstrom m, d.h. ein konstanter Gasstrom ein. Auch die Drehzahl U/min des Motors 27 und somit des Kompressors 20 bleibt relativ konstant.

In der Kombination der Betriebsphasen A und B steht dem Instrument 11 unabhängig von der Umgebungstemperatur und unabhängig von dem Druck des Gasspeichers 12 ein anwendungsgerechter Gasstrom praktisch sofort nach Aktivierung des Kryoinstruments 11, d.h. nach Betätigung des Aktivierungsschalters 34 zur Verfügung. Damit ist eine korrekte Biopsieentnahme und schnelles Ansprechen des Kryoinstruments 11 auch bei sehr schlanken Biopsiesonden möglich. Dies insbesondere ohne, dass der Kompressor vor der Aktivierung oder in Betriebspausen gewissermaßen vorsorglich laufen müsste und ohne dass irgendein Puffergefäß unter Druck gehalten werden müsste. So werden Geräuschentwicklung und Energieverbrauch sowie Gerätegewicht und -Volumen minimiert.

Bei der vorgenannten Ausführungsform wird der Kompressors 20 eingeschaltet, wenn der an dem Drucksensor S1 anliegende Druck P1 unter dem Mindestdruck Pmin liegt und er wird ausgeschaltet, wenn der Druck P1 bei oder über dem Maximaldruck Pmax liegt. Liegt der Druck P1 bei Aktivierungsbeginn dazwischen, wird der Kompressor 20 durchströmt, indem der Druck des Gases aus dem Gasspeicher 12 ausreicht, die Ventile des Kompressors 20 zu öffnen.

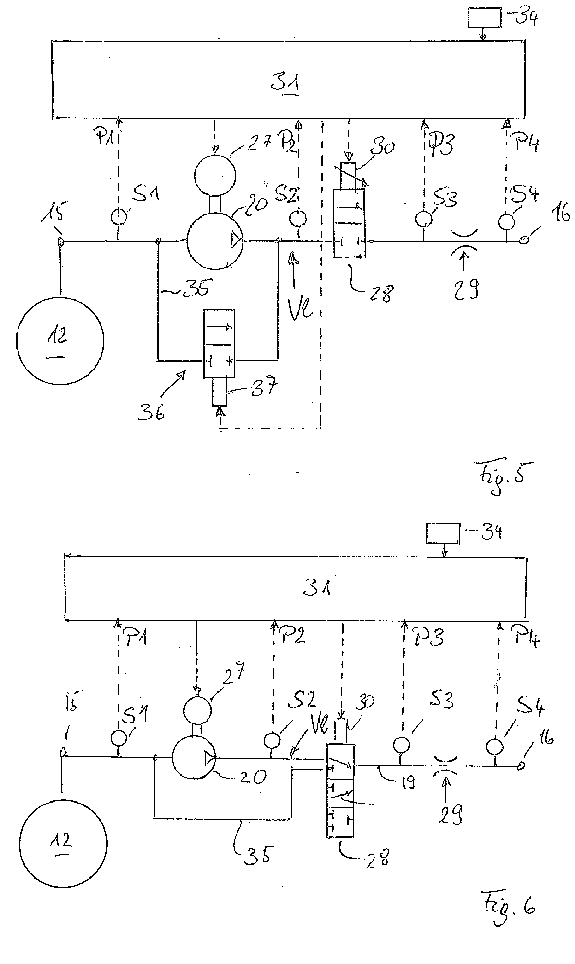

Die nachfolgend beschriebenen Ausführungsbeispiele (Figur 5 bis 8) vermeiden aber den dabei an diesen Ventilen auftretenden Druckverlust, indem für diese Betriebsart jeweils ein gesonderter Bypasskanal 35 vorgesehen ist.

Dazu veranschaulicht Figur 5 unter Zugrundelegung aller bisherigen bereits eingeführten Bezugszeichen und unter Zugrundelegung der zugehörigen Beschreibung ein Ausführungsbeispiel, bei dem der Bypasskanal 35 den Kompressor 20 überbrückt. In diesem Bypasskanal 35 ist ein Ventil, insbesondere ein 2/2-Wege-Ventil 36 angeordnet, das mittels eines Stellantriebs 37 in Schließposition oder Offenposition überführbar ist. Der Stellantrieb 37 ist dazu an die Steuereinrichtung 31 angeschlossen. Diese ist dazu eingerichtet, den Motor 27 stillzusetzen und das 2/2-Wege-Ventil 36 mittels des Stellantriebs 37 zu öffnen, wenn und solange der Druck P1 an dem Drucksensor S1 den Mindestdruck Pmin übersteigt.

Eine weitere Variante zeigt Figur 6, in der der Bypasskanal 35 innerhalb des Ventils 28 realisiert ist. Dieses ist dazu als 3/3-Wege-Ventil ausgebildet, bei dem in einer ersten Ventilstellung ein vollständiger Verschluss des Gaspfads 19 vorhanden ist, während in einer zweiten Ventilstellung der Bypasskanal 35 Teil des Gaspfads ist. In einer dritten Ventilstellung (in Figur 6 veranschaulicht) ist der Kompressor 20 Teil des Gaspfads).

Ansonsten gilt die vorige Beschreibung unter Zugrundelegung der bereits eingeführten Bezugszeichen entsprechend.

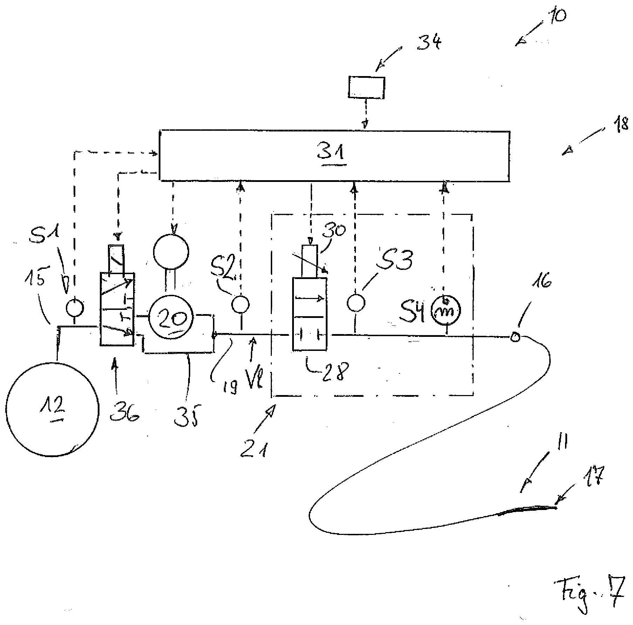

Eine abgewandelte Ausführungsform der erfindungsgemäßen Gasdosiereinheit 10 ist in Figur 7 veranschaulicht. Die Gasdosiereinheit 10 ist weitgehend mit der Gasdosiereinheit 10 nach Figur 5 identisch. Insoweit gilt die vorstehende Beschreibung unter Berücksichtigung der nachfolgenden Anmerkungen entsprechend: Der Sensor S4 ist als Massenflusssensor ausgebildet, der nicht zwangsläufig auf der Erfassung einer Druckdifferenz beruht. Es können andere Messprinzipien für Strömungssensoren Anwendung finden. Ein zweiter Unterschied liegt in dem Bypass-Ventil 36, das hier als 3/2-Wege-Ventil ausgebildet und zwischen dem Gasspeicher 12 und dem Kompressor 20 angeordnet ist. Es bildet eine Weiche, die den von dem Gasspeicher 12 herkommenden Gasfluss entweder zu dem Kompressor 20 oder über den Bypass 35 um diesen herum leitet. Das Ventil 36 ist von der Steuereinrichtung 31 gesteuert. Zur Funktion dieser Gasdosiereinheit 10 wird auf die Beschreibung der Ausführungsform nach Figur 5 verwiesen, die entsprechend gilt.

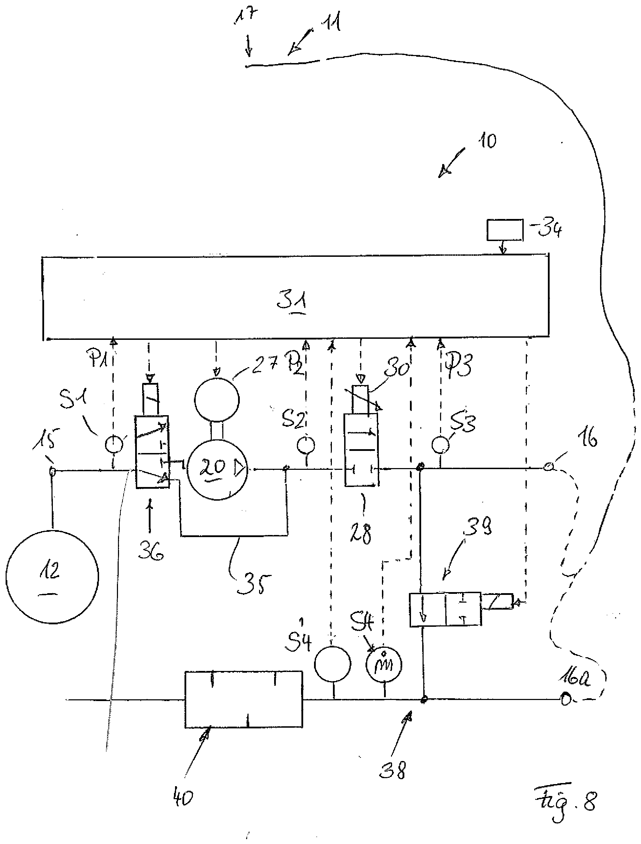

Eine weitere Ausführungsform der Gasdosiereinheit 10 geht aus Figur 8 hervor. Die dort veranschaulichte Gasdosiereinheit 10 basiert auf der Gasdosiereinheit nach Figur 7, deren Beschreibung hier entsprechend gilt. Im Unterschied zu den vorgeschriebenen Gasdosiereinheiten weist diese jedoch einen das Rückführungskanal 38 auf, an den eine Rückführungsleitung 16a des Instruments 11 anschließbar ist. Zwischen der zu dem Ausgangsanschluss 16 führenden druckführenden Leitung und dem Gasrückführungskanal 38 kann ein Entlüftungsventil 39 vorgesehen sein, dass wie alle anderen Aktoren auch, von der Steuereinrichtung 31 kontrolliert ist. Der Sensor S4 kann ein Massenstromsensor sein, der anders als bei den vorbeschriebenen Ausführungsformen nun in der Rückführungsleitung 38 angeordnet ist. Zumindest optional kann die Rückführungsleitung auch einen weiteren Sensor S4' umfassen. Beide Sensoren S4, S4' sind mit der Steuereinrichtung 31 verbunden. Außerdem kann in der Rückführungsleitung 38 ein Schalldämpfer 40 vorgesehen sein, der zu einem Gasausgang führt.

Ein solcher Gasrückführungskanal kann auch bei allen anderen Ausführungsformen nach Figur 2, 5, 6 oder 7 Anwendung finden. Außerdem ist es sinnvoll, die auf den Kompressor 20 folgenden Komponenten, insbesondere die Leitungen und Ventile 28, 39 und gegebenenfalls auch das Ventil 36, zu temperieren, insbesonder zu erwärmen, um ein Auskondensieren des komprimierten Gases zu verhindern. Dazu kann eine Erwärmungseinrichtung vorgesehen sein, die die betreffenden Komponenten einschließt. Mit oder ohne Temperiereinrichtung wird letztendlich eine Gasdosiereinheit 10 geschaffen, die in der Lage ist, auch bei kalten Gasflaschen, niedrigen Umgebungstemperaturen, fast leeren Gasflaschen sowie bei Verwendung sehr schlanker und/oder sehr langer Kryosonden korrekten Betrieb sicherzustellen. Solche Kryosonden können Längen von bis zu und über 2,55 m und einen Durchmesser von lediglich 1,1 bis hinunter zu 0,9 mm oder weniger aufweisen, wobei die erfindungsgemäße Gasdosiereinheit 10 in der Lage ist, die erhöhten Strömungswiderstände sicher zu überwinden. Solche Sonden eignen sich insbesondere zur Biopsieentnahme oder auch zur Behandlung von Gewebe in der Urologie, beispielsweise zur Biopsie, zur Diagnose verdächtiger Gewebe im oberen Harnweg und in der Pneumologie. Es eignet sich für miniaturisierte Bronchoskope mit Arbeitskanälen, die zum Beispiel lediglich 1,2 mm Lichtweite aufweisen. Demnach richtet sich die Erfindung auch auf die Gesamtheit, bestehend aus Instrument 11 und Gasdosiereinheit 10 sowie auf deren medizinische Anwendung zur Biopsieentnahme in der Urologie und/oder in der Pneumologie.

Die erfindungsgemäße Gasdosiereinheit 10 weist ein Druckregelmodul 18 mit einer Druckminderungseinrichtung 21 auf, die dazu eingerichtet ist, einen gewünschten Gasfluss m (insbesondere CO₂ Strom) einzustellen. Bei normaler Raumtemperatur reguliert die Steuereinrichtung 31 die Druckminderungseinrichtung 21 zur Einstellung eines gewünschten Drucks oder eines gewünschten Gasstroms. Ist die Raumtemperatur und/oder die Temperatur des angeschlossenen Gasspeichers 12 niedriger und reicht demzufolge der an dem Eingangsanschluss 15 der Gasdosiereinheit 10 anstehende Gasdruck für den korrekten Betrieb des Druckregelmoduls und des Instruments nicht aus, aktiviert die Steuereinrichtung einen in dem Gaspfad liegenden Kompressor 20, der den Druck vor dem Druckregelmodul 18 auf einen zum korrekten Betrieb des Druckregelmoduls 21 geeigneten Wert erhöht. Damit lässt sich das Kryoinstrument 11 unabhängig von der Raumtemperatur und unabhängig von dem Druck in dem Druckspeicher 12 korrekt aktivieren und benutzen. Sonst bestehende Betriebseinschränkungen werden vermieden.

### Bezugszeichen:

- 10: Gasdosiereinheit
- 11: Kryoinstrument
- 12: Gasspeicher
- 13: Anschluss des Gasspeichers
- 14: Leitung
- 15: Eingangsanschluss
- 16: Ausgangsanschluss
- 16a: Rückführungsanschluss
- 17: Spitze
- 18: Druckregelmodul
- 19, 19': Gaspfad
- Vl: Volumen des Gaspfads zwischen dem Kompressor 20 und dem Ventil 28
- 20: Kompressor
- 21: Druckminderungseinrichtung
- 22, 23: Zylinder
- 24, 25: Pleuelstanden
- 26: Kurbelwelle
- 27: Motor
- S1 ... S4: Drucksensoren S4'
- P1 ... P4: Drücke an den Drucksensoren S1 ... S4
- 28: Ventil
- 29: Drossel
- 30: Stellantrieb
- 31: Steuereinrichtung
- 32: Anzeigeeinrichtung
- 33: Eingabemittel
- 34: Aktivierungsschalter
- Pmin: Mindestdruck
- Pmax: Maximaldruck
- Psol: Solldruck
- m: Massenstrom
- 35: Bypasskanal
- 36: 2/2-Wegeventil
- 37: Stellantrieb
- 38: Gasrückführungskanal
- 39: Entlüftungsventil
- 40: Schalldämpfer

## Patentansprüche

1. Gasdosiereinheit (10) zur Gasversorgung eines Kryoinstruments (11),
mit einem Eingangsanschluss (15), an den ein Gasspeicher (12) anschließbar ist,
mit einem Ausgangsanschluss (16), an den ein Kryoinstrument (11) anschließbar ist,
mit einem zwischen dem Eingangsanschluss (15) und dem Ausgangsanschluss (16) angeordneten Druckregelmodul (18),
**dadurch gekennzeichnet,**
**dass** das Druckregelmodul (18) einen Kompressor (20), eine dem Kompressor (20) nachgeordnete Druckminderungseinrichtung (21) und eine Steuereinrichtung (31) aufweist, die dazu eingerichtet ist, den Kompressor (20) zu aktivieren, wenn ein vor der Druckminderungseinrichtung (21) zu messender Druck (P2) einen Grenzwert (Pmin) unterschreitet.

2. Gasdosiereinheit nach Anspruch 1, **dadurch gekennzeichnet, dass** der Kompressor (20) von einem Motor (27) angetrieben ist, der mit der Steuereinrichtung (31) verbunden ist, die dazu eingerichtet ist, den Motor (27) mit einer variablen, von ihr vorgegebenen Drehzahl zu betreiben.

3. Gasdosiereinheit nach einem der vorstehenden Ansprüche, **dadurch gekennzeichnet, dass** zwischen dem Eingangsanschluss (15) und dem Kompressor (20) ein erster Drucksensor (S1) angeordnet ist, der mit der Steuereinrichtung (31) verbunden ist, wobei die Steuereinrichtung (31) dazu eingerichtet ist, den Druck (P1) in einem angeschlossenen Gasspeicher (12) zu überwachen.

4. Gasdosiereinheit nach einem der vorstehenden Ansprüche, **dadurch gekennzeichnet, dass** zwischen dem Kompressor (20) und der Druckminderungseinrichtung (21) ein Drucksensor (S2) angeordnet ist, der mit der Steuereinrichtung (31) verbunden ist.

5. Gasdosiereinheit nach Anspruch 4, **dadurch gekennzeichnet, dass** die Steuereinrichtung (31) dazu eingerichtet ist, eine Steuerbetriebsart einzunehmen, in der sie den Motor (27) für eine vorgegebene Zeitspanne oder wenigstens so lange mit einer festgelegten Drehzahl (u) betreibt bis der zwischen dem Kompressor (20) und der Druckminderungseinrichtung (21) zu messende Druck (P2) einen festgelegten Mindestdruck (Pmin) überschritten, vorzugsweise einen Maximaldruck (Pmax) erreicht hat.

6. Gasdosiereinheit nach Anspruch 4 oder 5, **dadurch gekennzeichnet, dass** die Steuereinrichtung (31) dazu eingerichtet ist, eine Reglerbetriebsart einzunehmen, in der sie die Drehzahl (u) des Motors (27) entsprechend des mit dem Drucksensor (S2) gemessenen Drucks (P2) regelt.

7. Gasdosiereinheit nach einem der vorstehenden Ansprüche, **dadurch gekennzeichnet, dass** die Druckminderungseinrichtung (21) ein Ventil (28) enthält, das zwischen dem Kompressor (20) und dem Ausgangsanschluss (16) angeordnet ist.

8. Gasdosiereinheit nach Anspruch 7, **dadurch gekennzeichnet, dass** das Ventil (28) einen Stellantrieb (30) enthält und eine Offenstellung sowie eine Schließstellung aufweist.

9. Gasdosiereinheit nach Anspruch 8, **dadurch gekennzeichnet, dass** das Ventil (28) und der Stellantrieb (30) dazu eingerichtet ist, in Abhängigkeit von Stellsignalen der Steuereinrichtung (31) Zwischenstellungen zwischen der Offenstellung und der Schließstellung einzunehmen.

10. Gasdosiereinheit nach einem der vorstehenden Ansprüche 7 bis 9, **dadurch gekennzeichnet, dass** die Steuereinrichtung (31) mit einem Aktivierungsschalter (34) verbunden und dazu eingerichtet ist, den Kompressor (20) stillzusetzen und das Ventil (28) in Schließstellung zu überführen, wenn der Aktivierungsschalter (34) nicht aktiviert ist.

11. Gasdosiereinheit nach einem der vorstehenden Ansprüche 7 bis 10, **dadurch gekennzeichnet, dass** dem Ventil (28) ein dritter Drucksensor (S3) zur Erfassung eines dritten Drucks (P3) und ein Massenstromsensor (S4) nachgeordnet sind, wobei der Massenstromsensor (S4) mit der Steuereinrichtung (31) verbunden sind, die dazu eingerichtet ist, das Ventil (28) so zu steuern, dass der Massenstrom (m) an dem Ausgangsanschluss (16, 16a) einen festgelegten Wert aufweist.

12. Gasdosiereinheit nach einem der vorstehenden Ansprüche, **dadurch gekennzeichnet,**
**dass** die Steuereinrichtung (31) dazu eingerichtet ist, den Kompressor (20) stillzusetzen, wenn der mit dem ersten Drucksensor (S1) erfasste erste Druck (P1) wenigstens so groß ist wie ein Maximaldruck (Pmax) ist, und/oder
**dass** die Steuereinrichtung (31) dazu eingerichtet ist, den Kompressor (20) stillzusetzen, wenn der mit dem zweiten Drucksensor (S2) erfasste zweite Druck (P2) wenigstens so groß ist wie ein Maximaldruck (Pmax) ist.

13. Gasdosiereinheit nach einem der vorstehenden Ansprüche, **dadurch gekennzeichnet,**
**dass** die Steuereinrichtung (31) dazu eingerichtet ist, den Kompressor (20) mit einem Bypasskanal (35) zu überbrücken, wenn der mit dem ersten Drucksensor (S1) erfasste Pruck (P1) größer als ein Mindestdruck (Pmin) ist, und/oder
**dass** die Steuereinrichtung (31) dazu eingerichtet ist, den Kompressor (20) mit einem Bypasskanal (35) zu überbrücken, wenn der mit dem zweiten Drucksensor (S2) erfasste Pruck (P2) größer als ein Mindestdruck (Pmin) ist.

14. Gasdosiereinheit nach einem der vorstehenden Ansprüche, **dadurch gekennzeichnet, dass** der Kompressor (20) wenigstens zwei Zylinder (22, 23) mit gegenläufigen Kolben aufweist.

15. Gasdosiereinheit nach einem der vorstehenden Ansprüche, **dadurch gekennzeichnet, dass** der Kompressor (20) ein Hubvolumen (Vk) und zwischen dem Kompressor (20) und der Druckminderungseinrichtung (21) eine Leitung (19') mit einem Leitungsvolumen (Vl) vorgesehen sind, wobei das Verhältnis aus Hubvolumen (Vk) und Leitungsvolumen (Vl) größer als 1:5 ist.
